# EUROPEAN PATENT APPLICATION

(11) **EP 4 732 877 A1**
(43) Date of publication of application: **29.04.2026**
(21) Application number: 25210500.2
(22) Date of filing: 22.10.2025
(51) Int. Cl.: A61M 25/00, A61M 25/01, A61F 2/966, A61F 2/95, B29C 48/09, B29C 48/153, B29C 48/18, B29C 48/21

(54) **STENT DELIVERY SYSTEM**

(30) Priority: 22.10.2024 US 202463710168 P; 05.12.2024 US 202418970095
(71) Applicant: Micro Medical Solutions, Inc., Woburn, MA 01801 (US)
(72) Inventor: LABRECQUE, Brendan P., Woburn, MA 01801 (US); PAPPAS, Nikolaos D., Woburn, MA 01801 (US); SULLIVAN, Gregory, Woburn, MA 01801 (US)
(74) Representative: Schlich

(57) **Abstract**

Disclosed is a stent delivery system, including a delivery sheath catheter, a stabilizer, and a tuohy borst sideport. The delivery sheath catheter includes an inner lubricious liner, a reinforcement layer, and a wear-resistant outer jacket with an atraumatic distal tip, and a distal radiopaque marker band to facilitate visualization under fluoroscopy. The stabilizer, internally concentric within the delivery sheath catheter, includes an inner lumen for a 0.014-inch guidewire, a flat face for stent deployment, a proximal radiopaque split marker band to facilitate visualization under fluoroscopy, and a reinforced shaft. The preloaded stent is positioned between the delivery sheath catheter inner liner and the stabilizer lumen at the distal end of the delivery system. The stent delivery system, with a crossing profile of 3.20 to 3.89 French and a length between 40 to 220 cm, is configured for navigation through tight diseased vasculature to deploy the stent at distal lesion sites.

## Description

### BACKGROUND OF THE INVENTION

### Field of the Invention

The invention relates to medical devices, specifically to a minimally invasive stent delivery system designed for percutaneous access, trackability, and deployment into diseased vascular anatomies. More particularly, the invention pertains to a low crossing profile stent delivery system configured to navigate tight and tortuous arterial or venous pathways to deliver a self-expanding stent to distal lesion sites. The stent delivery system incorporates features to enhance flexibility, trackability, and precise placement in challenging anatomical conditions.

### Description of the Related Art

Need for medical intervention:
Within the body, a stenosis or abnormal narrowing may occur in a blood vessel or other tubular organ or structure. In a blood vessel, a stenosis or dissection from prior device use such as a balloon angioplasty, may obstruct blood. Within the art, stent devices may be used to treat stenoses. This invention is applicable to the delivery of such devices.

### Vascular stents:

A vascular stent is a small, mesh-like tube that is placed into a stenosed blood vessel to keep it open and restore blood flow. The device acts as a scaffold, supporting the vessel walls and preventing them from collapsing or otherwise causing obstruction. There exist a wide variety of vascular stents used for various applications. Vascular stents are typically deployed under fluoroscopy using stent delivery system catheters. Such a stent delivery system often includes a delivery sheath catheter and an interior stabilizer (pusher). Once placed, stents may be either self-expanding or balloon expandable. Self-expanding stents are often made from super elastic nitinol tubes or wire and utilize the inherent spring-like properties that can undergo large strains without permanent deformation when displacing from a loaded compressed state while in the stent delivery system and self-expanding during deployment inside a diseased vessel. In contrast, balloon-expandable stents require inflation of an integrated balloon to achieve their deployed configuration.

The design of the delivery sheath catheter is critical to the performance of a stent deployment catheter. A delivery sheath catheter is subject to a number of often conflicting design requirements such as flexibility, kink resistance, axial tensile and compressive strength, minimized exterior diameter, and maximized interior diameter. In particular, there is a balance between a need for flexibility and a need for strength. Therefore, a need remains for improved medical devices such as stent deployment catheters that are optimized for performance.

Unmet need(s) in the prior art:
Existing stent delivery systems face limitations in navigating extremely tight, tortuous, or distal vascular anatomies. These include inadequate balance between low profile and structural integrity, suboptimal friction management for stent loading and deployment. There remains a need for a stent delivery system that provides reliable low-profile tracking over standard 0.014 guidewires. Improved designs are sought to optimize these aspects, enabling safer, more efficient deployment of self-expanding stents in challenging anatomies.

In endovascular interventions, the Pin and Pull technique is sometimes used for deploying self-expanding stents. The stent is held in a compressed loaded state against the internal surface of an outer sheath and constrained in the interior by an inner catheter shaft. Retraction of the delivery sheath catheter via a pull-grip at the proximal end of the delivery sheath catheter while simultaneously keeping the inner shaft pinned" will result in stent deployment. Ancillary accessories are necessary to enable stent delivery system use, including guidewires ranging in diameter of 0.014", 0.018", or 0.035", as well as 4, 5, 6 or 7 French guiding sheath, and introducer sheaths.

### SUMMARY OF THE INVENTION

The invention, as illustrated herein, is clearly not anticipated, rendered obvious, or even present in any of the prior art mechanisms, either alone or in any combination thereof. A novel design is disclosed for a stent delivery system used with a preloaded self-expanding stent.

The invention pertains to a low crossing profile stent delivery system configured to navigate tight and tortuous arterial or venous pathways to deliver a self-expanding stent to distal lesion sites. The stent delivery system incorporates features to enhance flexibility, trackability, and precise placement in challenging anatomical conditions. The stent delivery system, with a crossing profile of 3.20 French to 3.89 French and a length between 40 to 220 cm, is configured for navigation through tight and tortuous diseased vasculature to deploy the stent at distal lesion sites reliably.

The stent delivery system described may be used to, but not limited to, deployment of the MicroStent, a self-expanding woven nitinol stent which is intended to maintain patency in vessels of 2.0 - 4.5 mm diameter, for lesions located below the knee having a length of 8mm to 119mm. The MicroStent is indicated for the treatment of critical limb threatening ischemia (CLTI) in the lower leg using the femoral or pedal artery as the interventional percutaneous access point.

The stent delivery system includes an outer delivery sheath catheter and interior stabilizer (pusher) catheter to hold a self-expanding stent in a loaded state. The delivery sheath catheter comprises a proximal hub, elongated shaft, and distal marker band. The proximal hub is used to flush and de-air the delivery sheath catheter prior to use. The elongated shaft allows the delivery sheath catheter to track over a guidewire to deliver the stent to the target lesion. The distal radiopaque marker band provides a visual reference under fluoroscopy for accurate placement of the stent.

The delivery sheath catheter offers the advantages of low profile, high tensile strength (for example in excess of, 20N), and good trackability for placement of vascular stents in small, tortuous blood vessels.

It is a further object of the present invention to provide a delivery sheath catheter with a low friction interior surface to allow for easier deployment of stents during medical procedures.

It is a further object of the present invention to provide a delivery sheath catheter and stabilizer configured to retain the stent in a loaded state, ready to use.

The stent delivery system is configured to maintain structural integrity and responsiveness during introductory forces, to maintain structural integrity and responsiveness while advancing percutaneously through a vascular anatomy, to maintain structural integrity and responsiveness during deployment of a preloaded stent at a target, and to maintain structural integrity and responsiveness during stent delivery system withdrawal from a patient.

A primary advantage to this stent delivery system is on low profile, while still being high-robustness and low elongation. High robustness is defined here as having a low chance of kinking when subjected to a 0.75" bend diameter, and if kinked not being damaged to the point being unusable, and abrasion resistant, and able to exceed 20N force without damage when loaded axially. Low elongation is described as both not exceeding 10% elongation at 10N of axial load, and being recoverable to original not loaded state (not sustaining deformation).

It is a further object of the present invention to provide a stabilizer catheter with an interior main lumen that is sufficiently robust, stiff and universally compatible with any 0.014" guidewire, of adequate length (for example, but not limited to 180cm length, 300cm length, etc.) having stock lubricous coatings (for example, water activated hydrophilic coatings, or dry lubricious PTFE coatings) for dependable stent delivery system tracking to a target distal lesion during medical procedures.

It is a further object of the present invention to provide a delivery sheath catheter and a stabilizer shaft that work together as system to maintain superior flexibility, kink resistance, and axial tensile and compressive strength when traversing tortuous paths during medical procedures.

It is a further object of the present invention to provide a delivery sheath catheter with a distal radiopaque distal marker band providing a visual reference of the distal position of the stent under fluoroscopy image guidance.

It is a further object of the present invention to provide a stabilizer catheter with a proximal radiopaque split marker band providing a visual reference of the proximal position of the stent under fluoroscopy image guidance.

It is a further object of the present invention to provide a delivery sheath catheter with a uniform stiffness, or a customizable stiffness profile along its length to allow for a multitude of clinical applications.

It is a further object of the present invention to provide a delivery sheath catheter with a soft distal region for increased maneuverability during medical procedures.

It is a further object of the present invention to provide a delivery system with a low overall crossing profile, to allow for easier maneuverability and increased usages during medical procedures.

There has thus been outlined, rather broadly, the more important features of a stent delivery system, the description thereof that follows may be better understood, and in order that the present contribution to the art may be better appreciated. There are additional features of the system that will be described hereinafter and which will form the subject matter of the claims appended hereto.

In this respect, before explaining at least one embodiment of the system in detail, it is to be understood that the system is not limited in its application to the details of construction, assembly and arrangements of the components set forth in the following description or illustrated in the drawings. The system is capable of other embodiments and of being practiced and carried out in various ways. Also, it is to be understood that the phraseology and terminology employed herein are for the purpose of description and should not be regarded as limiting.

These together with other objects of the system, along with the various features of novelty, which characterize the system, are pointed out with particularity in the claims annexed to and forming a part of this disclosure. For a better understanding of the system, its operating advantages and the specific objects attained by its uses, reference should be made to the accompanying drawings and descriptive matter in which there are illustrated preferred embodiments of the system.

The foregoing has outlined the more pertinent and important features of the present system in order that the detailed description of the system that follows may be better understood, and the present contributions to the art may be more fully appreciated. It is of course not possible to describe every conceivable combination of components and/or methodologies, but one of ordinary skill in the art may recognize that many further combinations or permutations are possible. Accordingly, the novel architecture described below is intended to embrace all such alterations, modifications, and variations that fall within the spirit and scope of the appended claims.

### BRIEF DESCRIPTION OF THE DRAWINGS

Advantages of the present system will be apparent from the following brief description of exemplary embodiments thereof; which description should be considered in conjunction with the accompanying drawings. Having thus described the invention in general terms, reference will now be made to the accompanying drawings, which are not necessarily drawn to scale. The system may be more completely understood in consideration of the following detailed description of various embodiments of the system in connection with the accompanying drawings, in which:
**FIG. 1** is a longitudinal side view of the stent delivery system with a preloaded self-expanding stent loaded at the distal end.
**FIG. 2** is a side cross sectional view of the stent delivery system distal end, showing the distal portion of the self-expanding stent present in the space allotted by the stabilizer and the delivery sheath.
**FIG. 3** is a side cross sectional view of the stent delivery system distal end, showing the proximal portion of the self-expanding stent present in the space allotted by the stabilizer and the delivery sheath catheter and abutting the stabilizer backstop feature.
**FIG. 4** is a side cross sectional view of the stent delivery system proximal end, showing the proximal end of the delivery sheath catheter **200** mounted permanently to the tuohy borst sideport **300,** and clamping to the exterior portion of the stabilizer **400.**
**FIG. 5** is a side cross sectional view of the stent delivery system proximal end, showing the stabilizer **400** proximal hub.
**FIG. 6A** is a perspective side view of stent delivery system with a stent loaded in the distal end of the stent delivery system.
**FIG. 6B** is a perspective side view of stent delivery system with a partially deployed from the distal end of the stent delivery system.
**FIG. 6C** is a perspective side view of stent delivery system with a stent deployed from the end.
**FIG. 7A** is a perspective side view of stent delivery system proximal end with a stent presumed to be loaded at the distal end.
**FIG. 7B** is a perspective side view of stent delivery system proximal end with a stent presumed to be deployed from the distal end.
**FIG. 8** is a perspective view of the various stent delivery system length embodiments.
**FIG. 9A** is a perspective view of delivery system sheath construction embodiment made with a solid super elastic Nitinol reinforcement layer.
**FIG. 9B** is a perspective view of delivery system sheath construction embodiment made a metallic hypotube having a spiral laser interruption cut reinforcement layer.
**FIG. 9C** is a perspective view of delivery system sheath construction embodiment made with a braided reinforcement layer.
**FIG. 10** is a perspective view of the various stent length and diameter embodiments.
**FIG. 11** is a perspective view of a stent delivery system fitted with an embodied braided delivery sheath catheter construction, showing robustness of the system.
**FIG. 12A** is a perspective view of a stent delivery system having a small crossing profile, allowing passage through an off-the-shelf 4 Fr Introducer Sheath.
**FIG. 13A** is a perspective view of an inner lumen preferred embodiment.
**FIG. 13B** is an exploded perspective view of the inner lumen preferred embodiment.
**FIG. 13C** is a perspective view of an inner lumen alternate embodiment.
**FIG. 13D** is an exploded perspective view of the inner lumen alternate embodiment.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the invention.

### DETAILED DESCRIPTION OF THE SEVERAL EMBODIMENTS

For the following defined terms, these definitions shall be applied, unless a different definition is given in the claims or elsewhere in this specification. All numeric values are herein assumed to be modified by the term "about", whether or not explicitly indicated. The term "about" generally refers to a range of numbers that one of skill in the art would consider equivalent to the recited value (*i.e.,* having the same function or result). In many instances, the term "about" may include numbers that are rounded to the nearest significant figure.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein reference numerals indicate like elements throughout the several views. The drawings, which are not necessarily to scale, depict illustrative embodiments of the claimed invention.

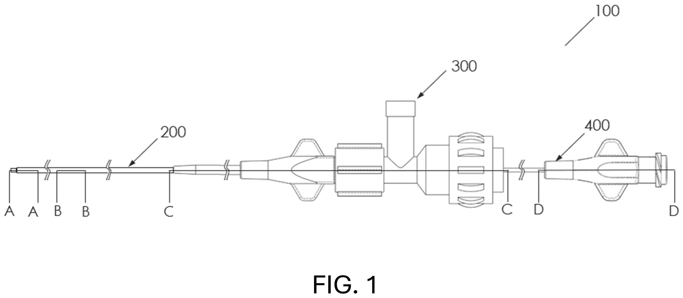

**FIG. 1** illustrates a side view of one embodiment of the stent delivery system **100** with a preloaded self-expanding **500** loaded at the distal end (not shown). The stent delivery system further comprises a delivery sheath catheter **200,** tuohy borst sideport **300,** and a stabilizer **400.** "Proximal" end refers to the end closer to an entry location outside the body, which is shown from the right side, whereas "distal" end refers to the farthest end from the entry location, and shown from the left side.

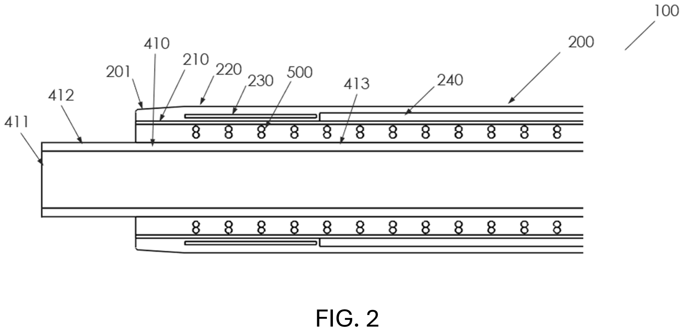

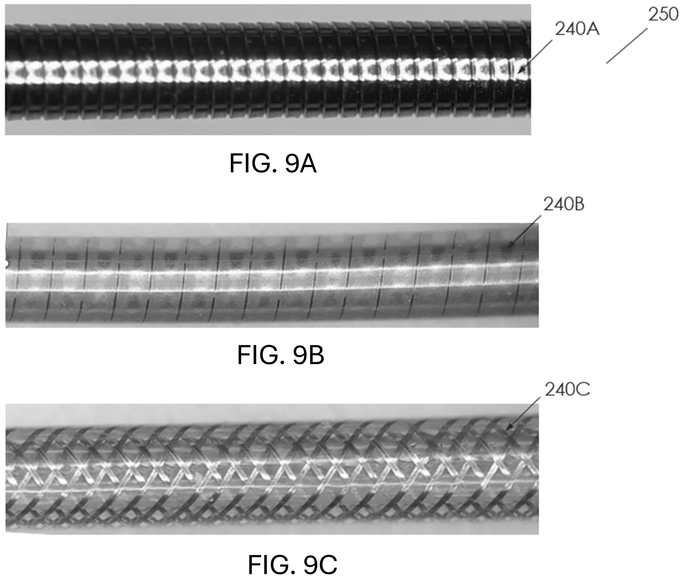

**FIG. 2** illustrates a side cross sectional view along line A-A of the stent delivery system **100** shown in **FIG. 1****,** showing the distal portion of the stent **500** in the loaded state within the space allotted by the stabilizer **400** and the delivery sheath catheter **200.** In one embodiment, the stabilizer **400** herein comprises an inner lumen **410,** which may be comprised of a distal cut polymer tube with a round open internal diameter of 0.0175"+/-0.0005" spanning the entire length of the stent delivery system interrupted. In one embodiment, the inner lumen **410** may have a wall thickness of 0.0025" and have a break strength of 20N or greater, and an elongation of less than 10% when axially loaded at 10N. In one embodiment, the inner lumen **410** may have an internal diameter that is concentric with the outside diameter by 90% or greater. In one embodiment, in order to ensure low friction passage of a 0.014" guidewire **700** (not shown), the inner lumen **410** is prepared at the distal tip having a clean square cut end **411.** In one embodiment, the inner lumen **410** has a protruded length **412** of 1mm to 3mm from the atraumatic tip **201,** to allow a clinician to locate and feed the stent delivery system **100** onto a 0.014" guidewire **700** (not shown). In one embodiment, the inner lumen **410** has an outer surface **413** that is abrasion resistant and supportive of the stent **500** in the loaded state. The inner lumen **410** extends the full length of the stent delivery system as a single, continuous, uninterrupted piece. The delivery sheath catheter **200** may comprise an atraumatic tip **201,** where only a lubricious inner liner **210** and soft wear resistant jacket polymer **220** laminated together are present. An atraumatic tip reduces forces on interior vessel walls during stent delivery system advancement to increase trackability. The stent **500** is loaded with inherent spring tension up against the lubricious inner liner **210,** and the edge is within the length of a radiopaque marker band **230.** In one embodiment the radiopaque marker band **230** is made of 90% platinum and 10% iridium and has an outside diameter the is coincident with the outside diameter of a reinforcement layer **240.** The delivery sheath catheter **200,** over the majority of the device length, comprises three layers: the lubricious inner liner **210,** the reinforcement layer **240,** and the soft wear resistant jacket polymer **220.** The lubricious inner liner **210** may be comprised of any suitable polymeric material. In various embodiments, the lubricious inner liner **210** may comprise a lubricious polymer such as polytetrafluoroethylene (PTFE) or fluorinated ethylene propylene (FEP), which may be etched to enhance thermal bonding to the shaft reinforcement layer **240.** In one embodiment, the lubricious inner liner **210** may be of 0.0010" ± 0.0005" thickness. In one embodiment, the lubricious inner liner **210** is thermally bonded to the reinforcement layer **240;** the reinforcement layer **240** would ideally have a polymer thermoplastic inner surface (see **FIG. 9B****),** or sufficient open wall (See **FIG. 9B** or **FIG. 9C**) allowing the soft wear resistant jacket polymer **220** to flow through the wall when melted during lamination at a temperature between 350F to 450F. The soft wear resistant jacket polymer **220** may be formed of any suitable polymeric material. In various embodiments, the soft wear resistant jacket polymer **220** may comprise a thermoplastic such as polyamide (*e.g.,* nylon), polyurethane (PU), polyethylene terephthalate (PET), polyether block amide (PEBA), and may be comprised of multiple durometers with the softest being at the atraumatic tip and the harder being more proximal. In one embodiment, the soft wear resistant jacket polymer **220** may be of 0.002" ± 0.001" thickness which may result in a final outside profile of 3.5 French (0.046"). In an additional embodiment, the soft wear resistant jacket polymer **220** may be even thicker to further improve durability/robustness, for example a soft wear resistant jacket polymer **220** thickness of 0.0045" may result in a final outside profile of 3.89 French (0.051"). In one embodiment the reinforcement layer **240** may be a single piece stainless steel or nitinol tube formed into a coil from 1 or more strands of wire; in a second embodiment the reinforcement layer **240** may be a single piece metal tube of either nitinol or stainless steel which is modified with a laser cut pattern through the wall to improve the flexibility; in a third embodiment the reinforcement layer **240** may be comprised of a 2 over 1 pattern metal braided structure with several triaxial fibers bundles running longitudinal woven between the diamonds of the braided wire.

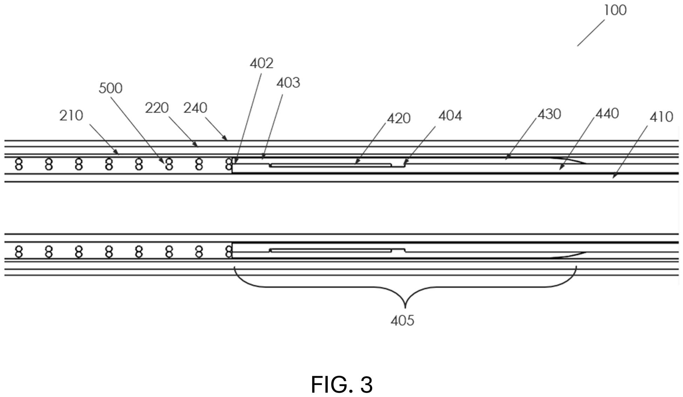

**FIG. 3** illustrates a side cross sectional view along line B-B of the stent delivery system **100** shown in **FIG. 1****,** showing the proximal portion of the self-expanding stent **500** present in the space allotted by the stabilizer **400** and the delivery sheath catheter **200.** The delivery sheath catheter **200** is shown to be consistent with its three layers: the lubricious inner liner **210,** the reinforcement layer **240,** and the soft wear resistant jacket polymer **220.** The stabilizer **400** herein comprises a flat face **402** in which the proximal end of stent **500** may bear load against, especially during deployment. Furthermore, the stabilizer **400** contains a collar length **403** that provides compressive load support during stent deployment, a radiopaque split marker band **420,** that may be embedded into shaft recess **404** formed into reinforced shaft **440** and is encapsulated by a tip jacket **430.** In one embodiment the radiopaque split marker band **420** is comprised of 90% platinum and 10% iridium. It is important to maintain low sliding forces, thus in one embodiment, the tip jacket **430** is smooth, cylindrical, and a low friction polymer. In one embodiment, the radiopaque split marker band **420** is cylindrical with a longitudinal cut through the wall of the part, allowing it to be assembled from the side of the shaft recess **404.** The feature as a whole, made up flat face **402,** collar length **403,** radiopaque split marker band **420,** shaft recess **404,** and tip jacket **430,** located over the tip jacket **430** is herein referred to as the stabilizer intermediate tip **405.** In one embodiment, the reinforced shaft **440** may be braid reinforced with strands of 0.0005" x 0.0025" full hard **304** stainless steel flat wire arranged in a 90 pic per inch density woven to a 2 over 1 over 2 braid configuration, and embedded between an inner layer of stiff thermoset polymer such as polyimide and outer layer of stiff thermoset polymer such as polyimide; and the shaft recess **404** is created by laser ablation. The inner lumen **410** extends the full length of the stent delivery system as a single, continuous, uninterrupted piece.

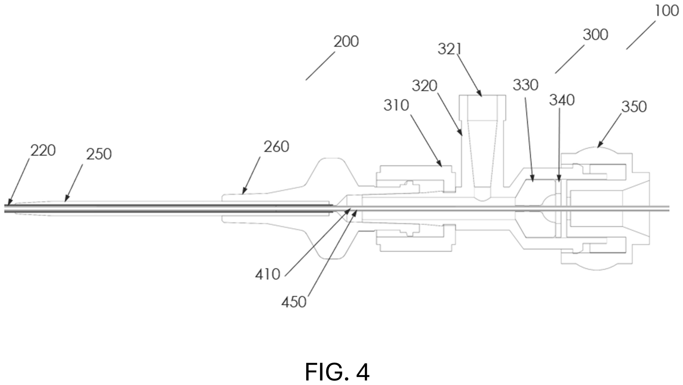

**FIG. 4** illustrates a side cross sectional view along line C-C of the stent delivery system **100** shown in **FIG. 1****,** containing the proximal side of the delivery sheath catheter **200** connected to the tuohy borst sideport **300,** and mounted over the stabilizer **400.** The soft wear resistant jacket polymer **220** terminates to a strain relief **250,** which contains a molded hub **260.** In one embodiment, the molded hub **260** with strain relief **250** are thermally bonded to the proximal end of the laminated shaft by connection to the soft wear resistant jacket polymer **220.** The stabilizer **400** consists of an inner lumen **410** and a metallic cannular **450** that concentrically pass from the center of the delivery sheath catheter **200** and into the touhy borst sideport **300,** where the metallic cannula **450** is sealed to a valve **330** before existing to open air. The tuohy borst sideport **300** comprises a tuohy body **320** that is mounted to a molded hub **260** by a Luer slip fit connection and held in place by a Luer thread between a nut **310** and the molded hub **260.** A rotating nut **350** is threaded to the tuohy body **320,** and may rotate to compress the valve **330,** which clamps to an immobilize metallic cannular **450.** The rotating nut **350** may be rotated in the opposite direction to allow movement of the metallic cannular **450.** A lubricious washer **340** translates rotating loads to compressive loads. In one embodiment, the valve **330** is a low durometer silicone rubber, of shore A 30A durometer. In one embodiment, the tuohy body **320** contains a female Luer bore **321** molded to 80369-7:2016 or ISO 594-1 standard, and is used for flushing the interior of the tuohy body **320,** and the space between the delivery sheath catheter **200** and the stabilizer **400,** including the stent **500** with normal 0.9% saline before use using an off the shelf syringe. The inner lumen **410** extends the full length of the stent delivery system as a single, continuous, uninterrupted piece.

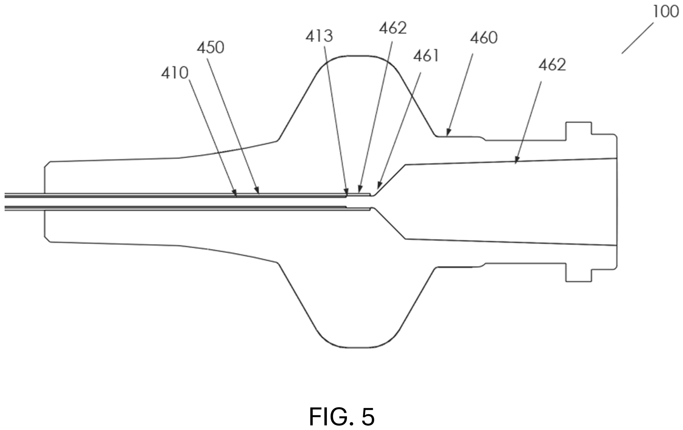

**FIG. 5** illustrates a side cross sectional view along line D-D of the stent delivery system **100** shown in **FIG. 1****,** showing the proximal portion of the stabilizer **400.** The inner lumen **410** and metallic cannular **450** are connected to a stabilizer molded hub **460.** In one embodiment, the cannula is grit blasted **462** to increase mechanical adhesion to the stabilizer molded hub **460** during an inert molding process and ensure a final tensile strength that exceeds 100N force. In one embodiment, in order to ensure low friction passage of a 0.014" guidewire **700** (not shown), the inner lumen **410** is prepared with a proximal clean square cut end **413;** and the stabilizer molded hub **460** has flash free smooth transition **461.** In one embodiment, the stabilizer molded hub **460** contains a female Luer bore **462** molded to 80369-7:2016 OR ISO 594-1 standard that is used for flushing the guidewire lumen of the stent delivery with normal 0.9% saline before use.

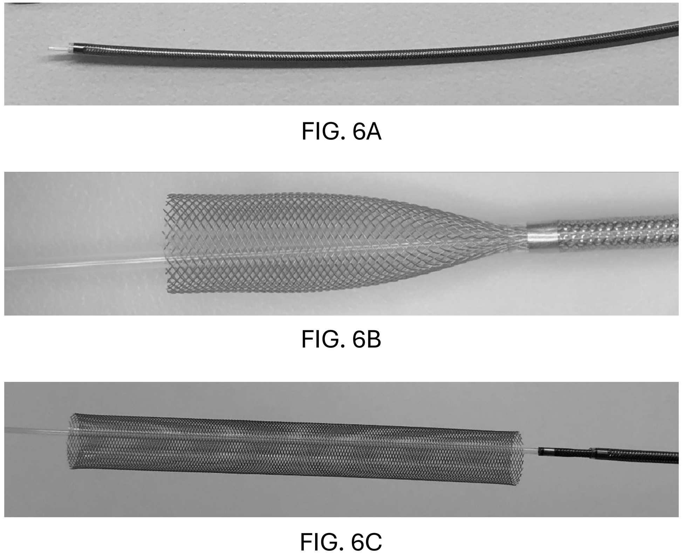

**FIG. 6A** is a perspective side view of the stent delivery system **100** shown in **FIG. 1****,** with a stent **500** loaded in the distal end of the stent delivery system. The stabilizer **400** protrudes from the delivery sheath catheter **200,** allowing it to be pushed onto a 0.014 in. guidewire.

**FIG. 6B** is a perspective side view of the stent delivery system **100** shown in **FIG. 1****,** and state of operation in which the stent **500** in the loaded state **FIG. 3A,** has deployed partially from the distal end of the stent delivery system.

**FIG. 6C** is a perspective side view of the stent delivery system **100** shown in **FIG. 1****,** with a stent **500** deployed from the end after having been loaded **FIG. 3A** and deployed partially **FIG. 3B.**

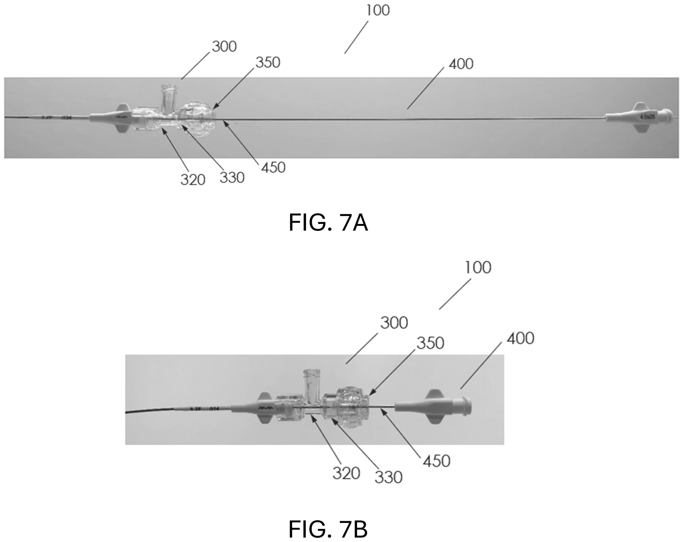

**FIG. 7A** is a perspective side view of the stent delivery system **100** shown in **FIG. 1****,** proximal end with a stent **500** presumed to be loaded at the distal end. The touhy borst sideport **400** is shown in a state where the rotating nut **350** is threaded to the touhy body **320** in a tightened position, thus the valve **330** is compressed and holding the metallic cannular **450** in a clamped position.

**FIG. 7B** is a perspective side view of the stent delivery system **100** shown in **FIG. 1****,** proximal end with a stent **500** presumed to be deployed at the distal end. The touhy borst sideport **400** herein contains the rotating nut **350** threaded to the touhy body **320,** which may be loosened from a decompress valve **330** and freeing metallic cannular **450** to translate. A motion will transfer compressive load to the stent, deploying it from the stent delivery system.

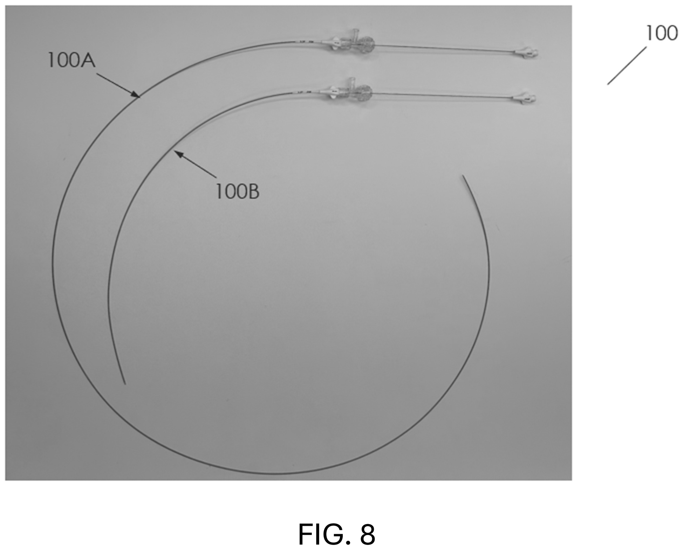

**FIG. 8** illustrates stent delivery system **100** length embodiments.

One stent delivery system **100** embodiment may be 120 CM length **100A.** A 120CM length may be intended to, but not limited to, accessing the bloodstream from a femoral access site and to navigate retrograde, over the lower arch, then antegrade through artery antegrade to below the knee sites.

One stent delivery system **100** embodiment may be 40CM length **100B.** A 40CM length may be intended to, but not limited to, accessing the bloodstream from the foot and to navigate through artery retrograde to below the knee sites.

One stent delivery system **100** embodiment may be 220 CM length to enable femoral contralateral and reach distal lesions in the food below the knee.

One Stent Delivery System **100** embodiment may be 150 CM length to enable femoral retrograde and reach lesions in the brain.

**FIG. 9A** illustrates a delivery system sheath **200** embodiment where the reinforcement layer **240** is made of a stainless steel or nitinol tube formed into a coil from 1 or more strands of wire (240A). The reinforcement layer **240A** is low thickness (between 0.0015-0.0020 thick), while still being high robustness.

**FIG. 9B** illustrates a delivery system sheath **200** embodiment where the reinforcement layer **240** is constructed of laser but hypotube (240B); further described as single piece metal tube of either super elastic nitinol or stainless steel which is modified with a laser cut spiral interrupt pattern through the wall to relieve stiffness and improve the flexibility. The reinforcement layer **240B** is low thickness (between 0.0015-0.0020 thick), while still being high robustness.

**FIG. 9C** illustrates a delivery system sheath **200** embodiment where the reinforcement layer **240** is made of a braided skeleton tube (240C), wherein metal wires are oriented helically in a braided 2 over 1 pattern structure with triaxial polymer fiber bundles oriented axially, running longitudinal between the diamonds of the braided wire; a pair of polymer layers comprises a first polymer layer which encapsulates the inside surface of the braided skeleton tube, and wherein a second polymer layer encapsulates the outside surface of the braided skeleton tube. The reinforcement layer **240C** is low thickness (between 0.0015-0.0020 thick), while still being high robustness.

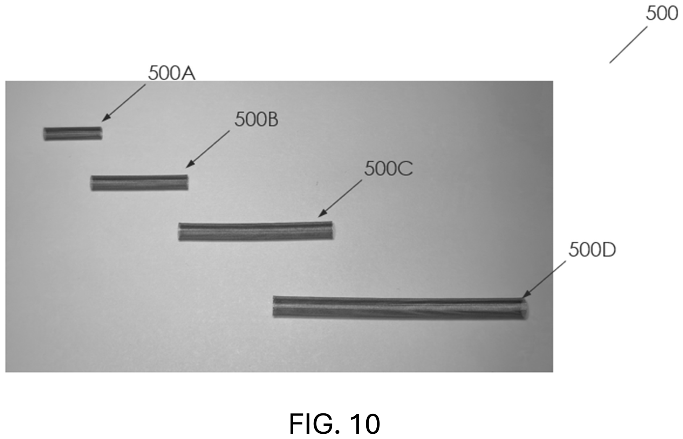

**FIG. 10** illustrates various stent **500** diameter and length embodiments arranged on a flat surface: 3.0mm diameter x 15mm long stent **500A,** 3.5mm diameter x 25mm long stent **500B,** 4.0mm diameter x 40mm long stent **500C,** 4.5mm diameter x 60mm long stent **500D.** The stent diameter and length have varying loaded state lengths, which in various embodiments differ by the final lengths of the reinforced shaft **440.**

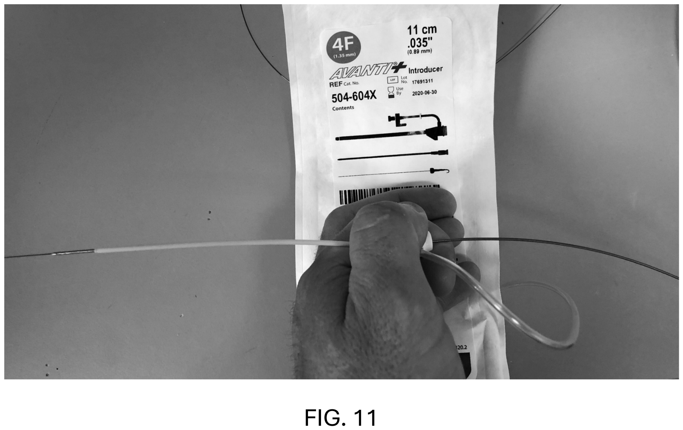

**FIG. 11** is a perspective view of a stent delivery system, where the small crossing profile is sufficiently smaller than the internal diameter of an off-the-shelf 4 Fr Introducer Sheath allowing free passage.

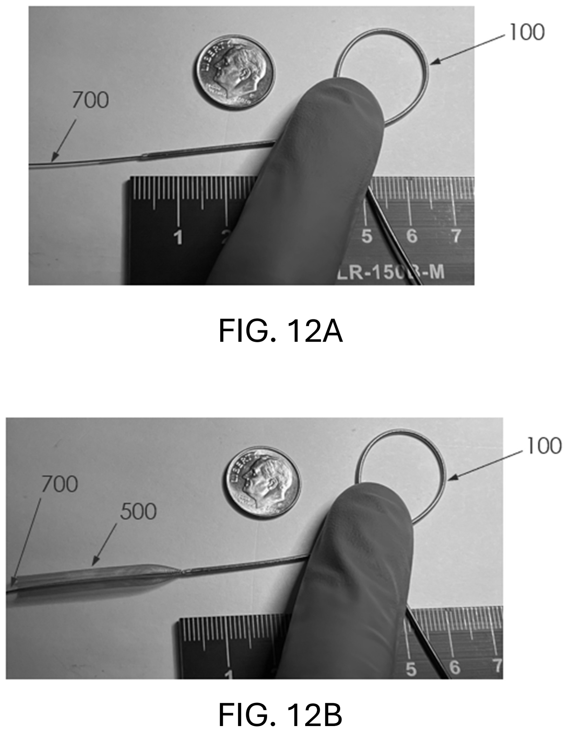

**FIG. 12A** is a perspective view of a stent delivery system **100** over a 0.014 guidewire **700,** showing robustness and durability of the system being flexed aggressively to a diameter of 0.75 inches (19mm), similar to the outside diameter of a U.S. dime, without sustaining damage such as kinking.

**FIG. 12B** is a perspective view of a stent delivery system **100** over a 0.014 guidewire **700,** showing robustness and durability of the system being flexed aggressively to a diameter of 0.75 inches (19mm), similar to the outside diameter of a U.S. dime, while reliably and dependably deploying a preloaded stent **500.**

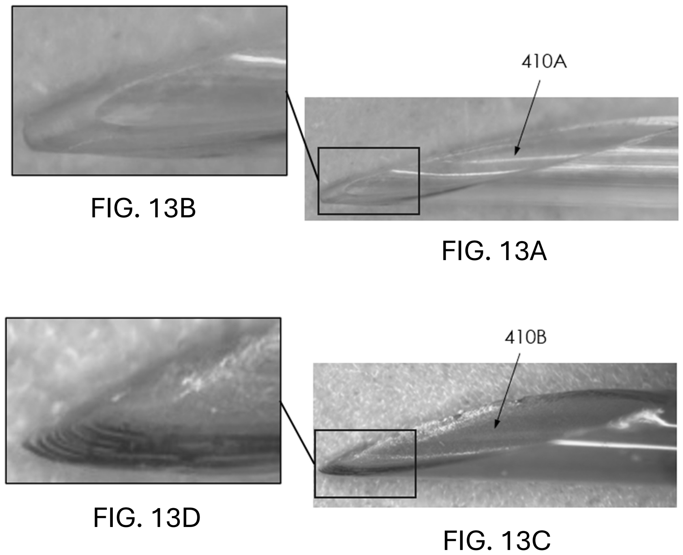

**FIGS. 13A-13B** show a perspective view of an inner lumen **410** of a preferred embodiment **410A,** cut at 60° angle to show a singular solid-cross-section of a homogeneous thermoplastic polymer.

**FIGS. 13C-13D** show a perspective view of an inner lumen **410** of an alternative embodiment **410B,** cut at 60⁰ angle to show a layered cross-section of a dispensed thermoset polymer (e.g. polyimide).

While the system is amenable to various modifications and alternative forms, specifics thereof have been shown by way of example in the drawings and will be described in detail. It should be understood, however, that the intention is not to limit the device to the particular embodiments described. On the contrary, the intention is to cover all modifications, equivalents, and alternatives falling within the spirit and scope of the system.

## Claims

1. A stent delivery system comprised of:
(a) a robust delivery sheath catheter comprising: a lubricious inner liner formed of a lubricious polymeric material; a reinforcement layer configured to enhance flexibility and structural integrity; and an outer jacket formed of a wear-resistant polymeric material, the outer jacket including an atraumatic tip at the distal end;
(b) a stabilizer comprising: a robust inner lumen configured to receive a guidewire having an inner diameter of approximately 0.0175 inches to fit freely with an 0.014 guidewire, the inner lumen extending from the proximal end to the distal end of the delivery sheath; and intermediate tip containing a flat face configured to bear a load of a preloaded stent during deployment; and a radiopaque split marker band embedded in a shaft recess of a stabilizer, and a radiopaque marker band being cylindrical with a longitudinal cut for side assembly;
(c) a self-expanding stent preloaded in a compressed state within a space defined between the lubricious inner liner of the delivery sheath catheter and the outer surface of inner lumen of the stabilizer, the stent positioned adjacent to a distal radiopaque marker band on the delivery sheath; and
(d) a tuohy borst sideport coupled to the proximal end of the delivery sheath, the sideport comprising: a body mounted to a molded hub of the delivery sheath catheter via a Luer slip fit connection; and a valve configured to selectively immobilize a metallic cannula of the stabilizer, the metallic cannula extending through the inner lumen and sealed to the valve;
wherein the system has a crossing profile between 3.20 Fr and 3.89 Fr and a length between 40cm and 220cm, and is configured to maintain structural integrity and responsiveness during introductory forces, to maintain structural integrity and responsiveness while advancing percutaneously through a vascular anatomy, to maintain structural integrity and responsiveness during deployment of a preloaded stent at a target, and to maintain structural integrity and responsiveness during stent delivery system withdrawal from a patient.

2. The stent delivery system of claim 1 consisting of an inner lumen material that is comprised of a singular solid cross-section of a homogeneous thermoplastic polymer, not a layered cross-section dispensed thermoset polymer (e.g. polyimide).

3. The stent delivery system of claim 1 comprising an inner lumen geometry that:
(a) has a generally circular internal open shape, with an internal diameter of 0.0175 inches ± 0.0005 inches, and a concentricity of at least 90% relative to its outer surface; and
(b) has an internal diameter compatible with a standard 0.014" guidewire or an internal diameter configured to receive a 0.014"guidewire with minimal resistance and free movement.

4. The stent delivery system of claim 1 comprising an inner lumen construction that:
(a) extends the full length of the stent delivery system as a single, continuous, uninterrupted piece;
(b) has clean square-cut ends; and
(c) has an outer surface along at least a portion of its length configured to support a stent in a compressed state.

5. The stent delivery system of claim 1 comprising a stabilizer intermediate tip containing:
(a) a flat face to bear compressive stress of a loaded state stent during deployment;
(b) a collar length that provides support to the flat face during stent deployment;
(c) a radiopaque split marker band cut through the wall of the part, allowing it to be assembled from the side of the shaft recess;
(d) encapsulation provided by a smooth, cylindrical, and a low friction thermoplastic polymer jacket; and
(e) a reinforced shaft, having specific length configurations relevant to both the length of the stent in the loaded state, and to the stent overall delivery system length.

6. The stent delivery system of claim 1 comprising a delivery sheath catheter comprised of:
(a) a low-thickness, high-robustness reinforcement layer;
(b) a lubricious inner liner that is thermally bonded to the reinforcement layer;
(c) a wear-resistant polymer jacket thermally bonded to the reinforcement layer;
(d) a laminated shaft structure in which the reinforcement layer, lubricious liner, wear-resistant polymer jacket, and a radiopaque marker band are thermally laminated together using a fluorinated ethylene propylene (FEP) shrink tube and heat exposure, the laminated shaft being configurable according to stent delivery system length specifications;
(e) hub with strain relief thermally bonded to the proximal end of the laminated shaft; and
(f) a radiopaque marker band positioned at the distal end of the delivery sheath catheter, the marker band having an outer diameter coincident with the outer diameter of the reinforcement layer.
